# EUROPEAN PATENT SPECIFICATION

(11) **EP 3 789 072 B1**
(45) Date of publication and mention of the grant of the patent: **30.10.2024**
(21) Application number: 18917477.4
(22) Date of filing: 01.05.2018
(51) Int. Cl.: A61M 25/09

(54) **GUIDE WIRE**
FÜHRUNGSDRAHT
FIL-GUIDE

(43) Date of publication of application: 10.03.2021
(73) Proprietor: ASAHI INTECC CO., LTD., Seto-shi, Aichi 489-0071 (JP)
(72) Inventor: KOSUGI Tomoki, Seto-shi, Aichi 489-0071 (JP); TAKAHASHI Daiki, Seto-shi, Aichi 489-0071 (JP)
(74) Representative: Winter, Brandl - Partnerschaft mbB
(86) International application number: PCT/JP2018/017468
(87) International publication number: WO 2019/211903

(56) References cited:
- EP-A1- 2 311 518
- EP-A1- 2 433 668
- EP-A1- 2 535 078
- WO-A1-2013/114985
- WO-A1-2018/062155
- JP-A- 2005 278 795
- JP-A- 2006 519 069
- JP-A- 2008 011 938
- JP-A- 2014 076 374
- JP-A- 2015 062 511
- JP-A- 2015 062 511
- JP-A- 2016 059 438
- JP-A- S51 136 979
- US-A1- 2008 281 396

## Description

### TECHNICAL FIELD

The disclosed embodiment relates to a guide wire.

### BACKGROUND ART

There is known a guide wire used for inserting a catheter into a blood vessel, a digestive organ, or the like. A guide wire made of a metal coil typically includes a core shaft using a wire material, and a coil body wound around an outer periphery of the core shaft, and a distal end of the core shaft and a distal end of the coil body are joined. In such a guide wire, a technique is known in which a resin layer is formed on the coil body for the purpose of improving slidability by smoothing the guide wire, thrombus adhesion preventing property, operability, and the like. For example, Patent Literatures 1, 2 and 3 disclose a guide wire in which a resin coating (resin layer) is formed on an outer periphery of a coil body.

### CITATION LIST

### Patent Literature

Patent Literature 1: Japanese Patent No. 5526218
Patent Literature 2: Japanese Unexamined Patent Application Publication No. 2008-237621
Patent Literature 3: JP 2015062511 A

However, the guide wire described in Patent Literature 1 has a problem that a resin layer is formed between a coil body and a core shaft without a gap, and thus, the flexibility of the guide wire is not sufficient. Further, the guide wire described in Patent Literature 2 has a problem that a resin layer may be peeled or damaged when a guide wire is curved. Specifically, when the guide wire is curved, a space between adjacent wires of a coil body expands at an outer side of the curve. In the guide wire described in Patent Literature 2, the resin layer is formed on an outer peripheral surface of the coil body, and thus, the thickness of the resin layer is reduced by the resin layer being elongated in a gap expanded between two adjacent lines of a wire, which may lead to peeling or damage of the resin layer.

The invention has been made to resolve the above-described problems, and an object thereof is to provide a technique of suppressing peeling and damage of a resin layer while flexibility is secured in a guide wire including a coil body and a resin layer.

### SUMMARY OF INVENTION

The invention is defined by the subject matter of the independent claim. Further aspects are disclosed in the subclaims. The invention has been made to resolve at least a part of the above-described problems, and can be implemented as the following aspects.
(1) According to one aspect of the invention, a guide wire is provided. The guide wire includes a core shaft, a coil body formed by winding a wire around the core shaft, and a resin layer covering at least an outer peripheral surface of the coil body. The coil body includes a sparsely wound part in which a gap is formed between adjacent wires, a void is formed between the coil body and the core shaft, the resin layer includes a protruding portion protruding toward the core shaft through the gap between the adjacent wires in the sparsely wound part, and the protruding portion extends toward the core shaft relative to an inner peripheral surface of the coil body, wherein the protruding portion contacts the core shaft.

In general, when the guide wire is curved, a wire forming a coil body moves with the curve to decrease (reduce) or enlarge (expand) a space between the adjacent wires. With this configuration, the guide wire includes the resin layer extending toward the core shaft relative to the inner peripheral surface of the coil body, and thus, the thickness of the resin layer in a gap expanded with the curve of the guide wire between the adjacent wires, can be kept thick as compared to, for example, a configuration in which the resin layer is formed only on the outer peripheral surface of the coil body. Therefore, with this configuration, it is possible to suppress peeling and damage of the resin layer between the adjacent wires when the guide wire is curved. Further, with this configuration, a void is formed between the coil body and the core shaft, and thus, the wire forming the coil body easily moves as compared to a configuration having no voids, such as a case where the resin layer is formed between the wire of the coil body and the core shaft without a gap. Therefore, with this configuration, it is possible to reduce power required to curve the guide wire and improve the flexibility of the guide wire.

In the guide wire according to the invention, the protruding portion contacts the core shaft. With this configuration, the resin layer contacts the core shaft, and thus, the thickness of the resin layer in a gap expanded with the curve of the guide wire between the adjacent wires, can be kept much thicker. Thus, it is possible to further suppress peeling and damage of the resin layer when the guide wire is curved.
(2) In the guide wire according to the invention, the protruding portion may be joined to the core shaft. With this configuration, the resin layer is joined to the core shaft, and thus, the thickness of the resin layer in a gap expanded with the curve of the guide wire between the adjacent wires, can be kept much thicker. Thus, it is possible to further suppress peeling and damage of the resin layer when the guide wire is curved.
(3) The guide wire according to the above aspect further may include a resin film covering an outer peripheral surface of the core shaft, and the protruding portion may be joined to the resin film. With this configuration, the guide wire includes the resin film covering the outer peripheral surface of the core shaft, and thus, the resin film provides smoothness to improve slidability of the guide wire and to prevent foreign matters such as thrombus from adhering to the outer peripheral surface of the core shaft.
(4) The guide wire according to the above aspect further may include an inner coil body formed by winding a wire around the core shaft at an inner side of the coil body. The inner coil body has a length shorter than a length of the coil body in an axial direction, and the sparsely wound part may be formed at a portion where the coil body does not cover the inner coil body. With this configuration, the sparsely wound part formed with the resin layer is located at a portion where the coil body does not cover the inner coil body, and thus, the movement of the inner coil body cannot be prevented by the resin layer. Thus, with this configuration, it is possible to maintain the flexibility of the guide wire even in the configuration including the inner coil body.

### BRIEF DESCRIPTION OF DRAWINGS

Fig. 1 is a schematic partial cross-sectional view illustrating an entire configuration of a guide wire according to a first example not forming part of the invention.
Fig. 2 is a partial enlarged view of the guide wire.
Fig. 3 is a diagram for explaining a resin layer when the guide wire is curved.
Fig. 4 is a diagram for explaining a resin layer when a guide wire in a comparative example is curved.
Fig. 5 is a partial enlarged view of a guide wire according to an embodiment of the invention.
Fig. 6 is a diagram for explaining a resin layer when the guide wire according to this embodiment of the invention is curved.
Fig. 7 is a partial enlarged view of a guide wire according to a further embodiment of the invention.
Fig. 8 is a diagram for explaining a resin layer when the guide wire according to the further embodiment of the invention is curved.
Fig. 9 is a schematic partial cross-sectional view illustrating an entire configuration of a guide wire according to an exampe not forming part of the invention.
Fig. 10 is a schematic partial cross-sectional view illustrating an entire configuration of a guide wire according to yet a further example not forming part of the invention.
Fig. 11 is a partial enlarged view of a guide wire according to a further embodiment of the invention.
Fig. 12 is a partial enlarged view of a guide wire according to yet a further example not forming part of the invention.
Fig. 13 is a partial enlarged view of a guide wire according to yet a further example not forming part of the invention.
Fig. 14 is a partial enlarged view of a guide wire according to a ninth embodiment.

### EMBODIMENTS OF THE INVENTION

### <First Embodiment (Reference Embodiment)>

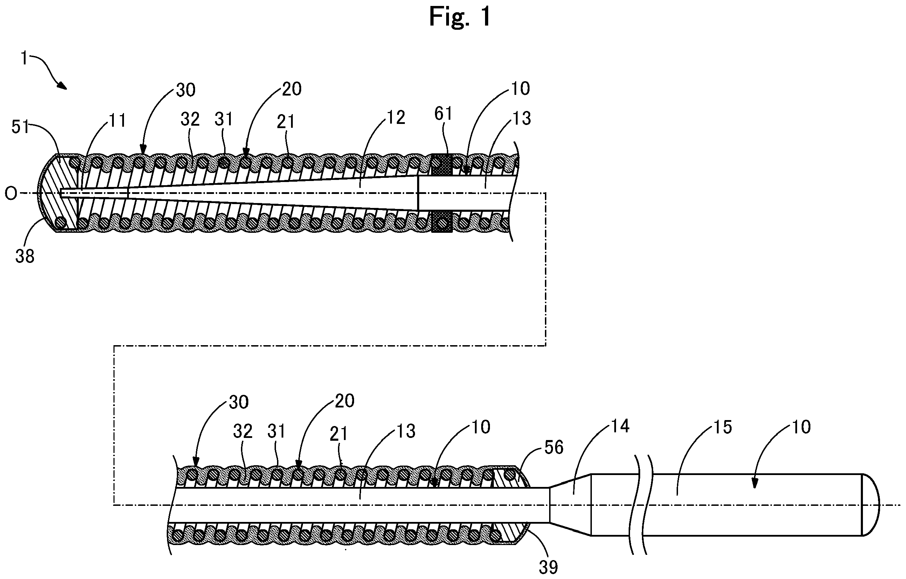

Fig. 1 is a schematic partial cross-sectional view illustrating an entire configuration of a guide wire 1 according to a first example. The guide wire 1 is a medical device used when a catheter is inserted into a blood vessel or a digestive organ, and includes a core shaft 10, a coil body 20, a resin layer 30, a distal end joint part 51, a proximal end joint part 56, and an intermediate fixing part 61.

In Fig. 1, an axis passing through the center of the guide wire 1 is represented by an axis O (dash-dot-dash line). The left side in Fig. 1 is referred to as a "distal end side" of the guide wire 1 and components, and the right side in Fig. 1 is referred to as a "proximal end side" of the guide wire 1 and components. Further, with respect to the guide wire 1 and components, an end part located on the distal end side is referred to as a "distal end part" or simply a "distal end," and an end part located on the proximal end side is referred to as a "proximal end part" or simply a "proximal end" . In the present embodiment, the distal end side corresponds to a "distal side," and the proximal end side corresponds to a "proximal side." These are common to the drawings illustrating the entire configuration after Fig. 1.

The core shaft 10 is a tapered long member having a large diameter at the proximal end side and a small diameter at the distal end side. The core shaft 10 may be formed of a material such as a stainless alloy (SUS304, SUS316, etc.), a superelastic alloy such as an Ni-Ti alloy, a piano wire, a nickel-chromium base alloy, a cobalt alloy, and tungsten. The core shaft 10 may be formed of a known material other than the above. The core shaft 10 includes a small-diameter part 11, a first tapered part 12, a first large-diameter part 13, a second tapered part 14, and a second large-diameter part 15 in this order from the distal end side to the proximal end side. An outer diameter and length at a portion of the core shaft 10 may be optionally determined.

The small-diameter part 11 is formed at the distal end part of the core shaft 10. The small-diameter part 11 is a part where the outer diameter of the core shaft 10 is smallest, and has a substantially cylindrical shape having a constant outer diameter. The distal end joint part 51 is formed at the distal end part of the small-diameter part 11. The first tapered part 12 is formed between the small-diameter part 11 and the first large-diameter part 13. The first tapered part 12 has a tapered shape whose outer diameter increases from the distal end side toward the proximal end side. The first large-diameter part 13 is formed between the first tapered part 12 and the second tapered part 14. The first large-diameter part 13 has a substantially cylindrical shape having a constant outer diameter larger than the outer diameter of the small-diameter part 11. The proximal end joint part 56 is formed at the proximal end part of the first large-diameter part 13. Outer peripheral surfaces of the small-diameter part 11, the first tapered part 12, and the first large-diameter part 13 are covered by the coil body 20 described later.

The second tapered part 14 is formed between the first large-diameter part 13 and the second large-diameter part 15. The second tapered part 14 has a tapered shape whose outer diameter increases from the distal end side toward the proximal end side. The second large-diameter part 15 is formed at the proximal end part of the core shaft 10. The second large-diameter part 15 is a portion where the outer diameter of the core shaft 10 is largest, and has a substantially cylindrical shape having a constant outer diameter. The second tapered part 14 and the second large-diameter part 15 are not covered by the coil body 20, and are used when an operator grasps the guide wire 1.

The coil body 20 has a substantially hollow cylindrical shape in which a wire 21 is spirally wound around the core shaft 10. The coil body 20 of the present embodiment is arranged to cover the outer peripheral surfaces of the small-diameter part 11, the first tapered part 12, and the first large-diameter part 13 of the core shaft 10, and is not arranged on the second tapered part 14 and the second large-diameter part 15 of the core shaft 10. The wire 21 forming the coil body 20 may be a solid wire composed of one wire or a strand obtained by twisting a plurality of wires. If the wire 21 is a solid wire, the coil body 20 is configured as a single coil, and if the wire 21 is a strand, the coil body 20 is configured as a hollow stranded coil. Further, the coil body 20 may be configured by combining a single coil and a hollow stranded coil. A wire diameter of the wire 21 and an average coil diameter in the coil body 20 (an average diameter of outer and inner diameters of the coil body 20) may be optionally determined.

The wire 21 may be formed of, for example, a stainless alloy (SUS304, SUS316, etc.), a superelastic alloy such as an Ni-Ti alloy, a piano wire, a nickel-chromium base alloy, a cobalt alloy, a radiolucent alloy such as tungsten, gold, platinum, tungsten, and a radiopaque alloy such as an alloy containing these elements (for example, a platinum-nickel alloy). The wire 21 may be formed of a known material other than the above.

The coil body 20 is fixed to the core shaft 10 by the distal end joint part 51, the proximal end joint part 56, and the intermediate fixing part 61. The distal end joint part 51 is a member that joins a distal end part of the coil body 20 and the distal end part of the small-diameter part 11 of the core shaft 10. The distal end joint part 51 is formed of metal solder such as silver solder, gold solder, zinc, an Sn-Ag alloy, and an Au-Sn alloy. The distal end of the coil body 20 and the distal end of the small-diameter part 11 are fixed by the metal solder. The distal end joint part 51 may be formed by an adhesive such as an epoxy-based adhesive.

The proximal end joint part 56 is a member that joins a proximal end part of the coil body 20 and the proximal end part of the first large-diameter part 13 of the core shaft 10. The proximal end joint part 56 may be formed of a material that is same as the material of the distal end joint part 51, or may be formed of a material that is different from the material of the distal end joint part 51. The intermediate fixing part 61 is a member that fixes the coil body 20 and the first large-diameter part 13 of the core shaft 10, in the vicinity of an intermediate portion of the coil body 20 in an axis O direction. The intermediate fixing part 61 may be formed of a material that is same as the materials of the distal end joint part 51 and the proximal end joint part 56, or may be formed of a material that is different from the materials of the distal end joint part 51 and the proximal end joint part 56. The guide wire 1 may include a plurality of fixing parts for fixing the coil body 20 and the core shaft 10.

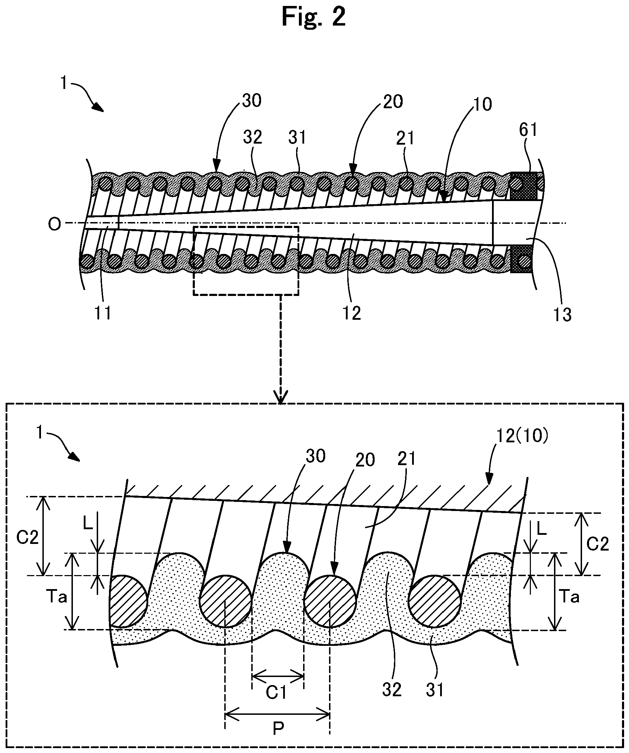

Fig. 2 is a partial enlarged view of the guide wire 1. The upper side in Fig. 2 illustrates a portion of the guide wire 1, from the small-diameter part 11 to the first large-diameter part 13. The lower side in Fig. 2 illustrates an enlarged view of a portion surrounded by a broken-line rectangle in the upper side. As with Fig. 1, the left side in Fig. 2 corresponds to the distal end side (distal side) of the guide wire 1 and components, and the right side in Fig. 2 corresponds to the proximal end side (proximal side) of the guide wire 1 and components. These are common to the partial enlarged views after Fig. 2.

In the coil body 20, gaps C1 are formed between the adjacent wires 21 over an entire region in the axis O direction (Fig. 1 and the lower side in Fig. 2). In the present embodiment, such a gap C1 is defined as a size (length) at a portion where a distance between the adjacent wires 21 is closest in any cross section in the axis O direction. The size of the gap C1 may be optionally determined. In the guide wire 1 according to the present embodiment, as illustrated in the lower side in Fig. 2, the gaps C1 formed between the adjacent wires 21 have a constant size equal to the wire diameter of the wire 21. All the gaps C1 may have the same size, at least some of the gaps C1 may have different sizes, or all of the gaps C1 may have different sizes.

Hereinafter, of the coil body 20, a portion having the gap C1 between the adjacent wires 21 is also referred to as a "sparsely wound part," and a portion having no gap C1 between the adjacent wires 21 (in other words, a portion where the adjacent wires 21 contact with each other without any gap) is also referred to as a "closely wound part." In the guide wire 1 according to the present embodiment, the coil body 20 includes the sparsely wound part over the entire region in the axis O direction.

In the coil body 20, a void C2 is formed between the outer peripheral surface of the core shaft 10 and an inner peripheral surface of the wire 21 of the coil body 20 (that is, an inner peripheral surface (inner surface) of the coil body 20) over the entire region in the axis O direction (Fig. 1 and the lower side in Fig. 2). In the present embodiment, the void C2 is defined as a size (length) at a portion where a distance between the inner peripheral surface of the wire 21 and the outer peripheral surface of the core shaft 10 is closest in any cross section in the axis O direction. The size of the void C2 may be optionally determined. In the guide wire 1 according to the present embodiment, the inner diameter of the coil body 20 is constant while the outer diameter of the core shaft 10 increases from the distal end side toward the proximal end side. Thus, as illustrated in the lower side in Fig. 2, the void C2 formed between the inner peripheral surfaces of the wire 21 and the outer peripheral surface of the core shaft 10 is gradually smaller from the distal end side to the proximal end side.

The resin layer 30 is a layer of resins that cover at least the outer peripheral surface (outer surface) of the coil body 20. The resin layer 30 may be formed of a resin material having hydrophobicity, a resin material having hydrophilicity, or a mixture thereof. Examples of the resin material having hydrophobicity to be employed include silicone resin, polyurethane, polyethylene, polyvinyl chloride, polyester, polypropylene, polyamide, polystyrene, polyolefin elastomer, polyester elastomer, polyamide elastomer, and polyurethane elastomer. Examples of the resin material having hydrophilicity to be employed include starch-based resin such as carboxymethyl starch, cellulose-based resin such as carboxymethylcellulose, polysaccharides such as alginic acid, chitin, chitosan, and hyaluronic acid, natural water-soluble polymer substances such as gelatin, and synthetic water-soluble polymer substances such as polyvinyl alcohol, polyethylene oxide, polyethylene glycol, polypropylene glycol, polyvinylpyrrolidone, and water-soluble nylon. The resin material having hydrophilicity becomes swollen when containing water, and thus, it is possible to improve slidability and thrombus adhesion preventing property as compared to the resin material having hydrophobicity.

The resin layer 30 includes a surface layer 31, protruding portions 32, a distal end resin layer 38, and a proximal end resin layer 39. The surface layer 31 is a part of the resin layer 30 and is a layered portion that covers the outer peripheral surface of the coil body 20. The shape of the surface layer 31 in the cross section illustrated in Fig. 2 is a wave shape. The thickness of the surface layer 31 may be optionally determined. The distal end resin layer 38 is a part of the resin layer 30 and is a layered portion that covers an outer peripheral surface of the distal end joint part 51. The proximal end resin layer 39 is a part of the resin layer 30 and is a layered portion that covers an outer peripheral surface of the proximal end joint part 56.

Such a protruding portion 32 is a part of the resin layer 30 and is a portion protruding toward the core shaft 10 through the gap C1 between the adjacent wires 21 of the coil body 20. The protruding portion 32 extends toward the core shaft 10 relative to the inner peripheral surface of the coil body 20, in other words, a surface of the wire 21 on a side of the core shaft 10. The shape of a protrusion of the protruding portion 32 in the cross section illustrated in Fig. 2 is an arc shape. In the present embodiment, a protruding length L of the protruding portion 32 is defined as a length between the surface of the wire 21 on the side of the core shaft 10 and a protrusion distal end of the protruding portion 32 (a portion closest to the core shaft 10) in any cross section in the axis O direction. The protruding length L may be optionally determined. In the guide wire 1 according to the present embodiment, as illustrated in the lower side in Fig. 2, the protruding lengths L of the protruding portions 32 formed between the adjacent wires 21 are constant (same length) and are set to about half of the wire diameter of the wire 21. At least some or all of the protruding lengths L of the protruding portions 32 may be different from each other.

In the present embodiment, a thickness Ta of the resin layer 30 is defined as a thickness (length) of the resin layer 30 in a middle part between the adjacent wires 21 in any cross section in the axis O direction. In the guide wire 1 according to the present embodiment, as illustrated in the lower side in Fig. 2, the thickness Ta of the resin layer 30 between the adjacent wires 21 is constant (same thickness) and is set to about 1.5 times to about 2 times the wire diameter of the wire 21. Further, in the present embodiment, all of the protruding portions 32 included in the resin layer 30 and the core shaft 10 are separated and not in contact. The thickness of the resin layer 30 may be optionally determined, and at least some or the thickness of the resin layer 30 at all or some portions may be different from each other.

The resin layer 30 may be formed, for example, by applying a liquidized resin material to the outer peripheral surface of the coil body 20. Specifically, the surface layer 31 is formed through the solidification of the resin material that adheres to the outer peripheral surface of the coil body 20. In addition, the protruding portion 32 is formed through the solidification of the resin material dropped toward the core shaft 10 from the gaps C1 between the adjacent wires 21 of the coil body 20. Thus, the resin layer 30 can be easily formed. In the present embodiment, the surface layer 31 and the protruding portion 32 are formed integrally, but the surface layer 31 and the protruding portion 32 may be formed separately. If the surface layer 31 and the protruding portion 32 are formed separately, for example, the protruding portions 32 may be formed by applying a liquidized resin material, and then the surface layer 31 may be formed by winding a thin film-like resin material around the outer peripheral surface of the coil body 20. If the surface layer 31 and the protruding portion 32 are formed separately, the surface layer 31 and the protruding portion 32 may be formed by using different resin materials. In addition, an extruder may be used to extrude a resin material, and then the extruded resin material may pass through the gaps C1 to form the protruding portion 32.

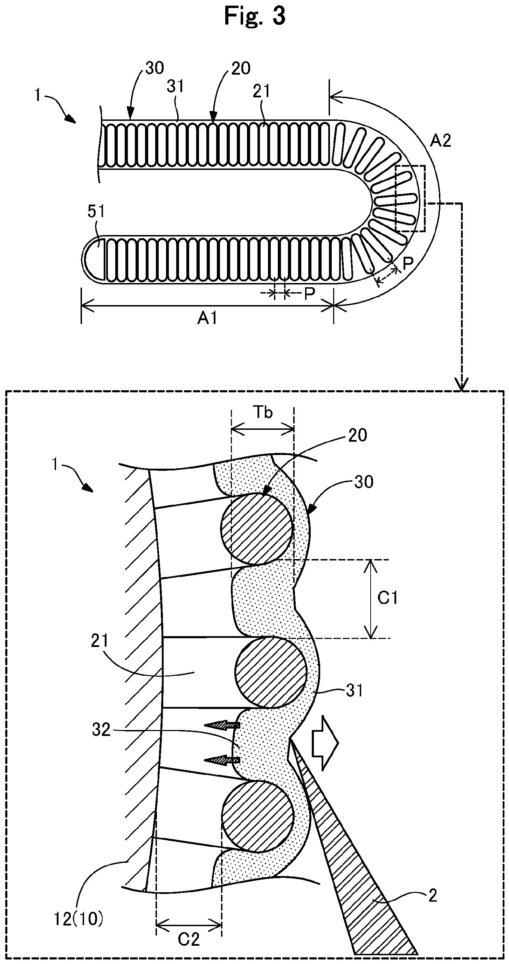

Fig. 3 is a diagram for explaining the resin layer 30 when the guide wire 1 is curved. The upper side in Fig. 3 illustrates a schematic diagram representing a portion at the distal end side of the curved guide wire 1. The lower side in Fig. 3 illustrates an enlarged view of a portion surrounded by a broken-line rectangle in the upper side. These are common to the explanatory diagrams after Fig. 3.

In general, if the guide wire 1 is curved, the wire 21 forming the coil body 20 moves with the curve to decrease (reduce) or enlarge (expand) a space P between the adjacent wires 21. Specifically, there is no change in the space P between the adjacent wires 21 in a region A1 having no curve. Therefore, the gap C1 between the adjacent wires 21 is as described with reference to Figs. 1 and 2. On the other hand, in a region A2 having the curve, the space P between the adjacent wires 21 changes with the curve to cause clogging or enlargement between the adjacent wires 21. With the change in the space P, the gap C1 between the adjacent wires 21 on an outer side of a curved part increases, and the gap C1 between the adjacent wires 21 on an inner side of the curved part decreases.

Here, the guide wire 1 according to the present embodiment includes the resin layer 30 (protruding portion 32) extending toward the core shaft 10 relative to the inner peripheral surface of the coil body 20. Thus, as illustrated in the lower side in Fig. 3, even if the gap C1 between the adjacent wires 21 expands on the outer side of the curved part, and with the expansion, the resin layer 30 is elongated, resulting in that the thickness of the resin layer 30 becomes thinner from a "thickness Ta during a normal time" to a "thickness Tb while being elongated", the thickness Tb of the resin layer 30 at the gap C1 expanded between the adjacent wires 21 can be kept thick. Thus, even if a distal end of an additional device 2 (a device such as a catheter used together with the guide wire 1) is caught on a surface of the resin layer 30, and a force is applied in a direction in which the resin layer 30 is peeled off from the coil body 20 (lower side in Fig. 3: white arrow), resistance to the peeling force (lower side in Fig. 3: arrow with oblique hatching) can be obtained. Consequently, in the guide wire 1 according to the present embodiment, it is possible to suppress peeling and damage of the resin layer 30 between the adjacent wires 21 when the guide wire 1 is curved.

In addition, the guide wire 1 according to the present example includes the resin layer 30 (protruding portion 32) extending toward the core shaft 10 relative to the inner peripheral surface of the coil body 20. Therefore, for example, even if the core shaft 10 is bent inside the coil body 20 when the guide wire 1 is used, the core shaft 10 comes into contact with the protruding portion 32 of the resin layer 30, and thus, is prevented from contacting with the coil body 20. The resin layer 30 (protruding portion 32) is formed of a resin material having flexibility as described above. Thus, in the guide wire 1 according to the present embodiment, damage and deformation of the core shaft 10 and the coil body 20 can be suppressed.

Further, in the guide wire 1 according to the present example, the void C2 is formed between the coil body 20 and the core shaft 10. Therefore, the wire 21 forming the coil body 20 easily moves as compared to, for example, a configuration having no voids C2 such as a case where the resin layer 30 is formed between the coil body 20 and the core shaft 10 without a gap. As a result, in the guide wire 1 according to the present embodiment, it is possible to reduce power required to curve the guide wire 1 and improve the flexibility of the guide wire 1. Further, in the guide wire 1 according to the present embodiment, the void C2 is formed between the coil body 20 and the core shaft 10, and thus, the resin layer 30 can be easily formed as compared to a configuration having no voids C2.

### <Comparative Example>

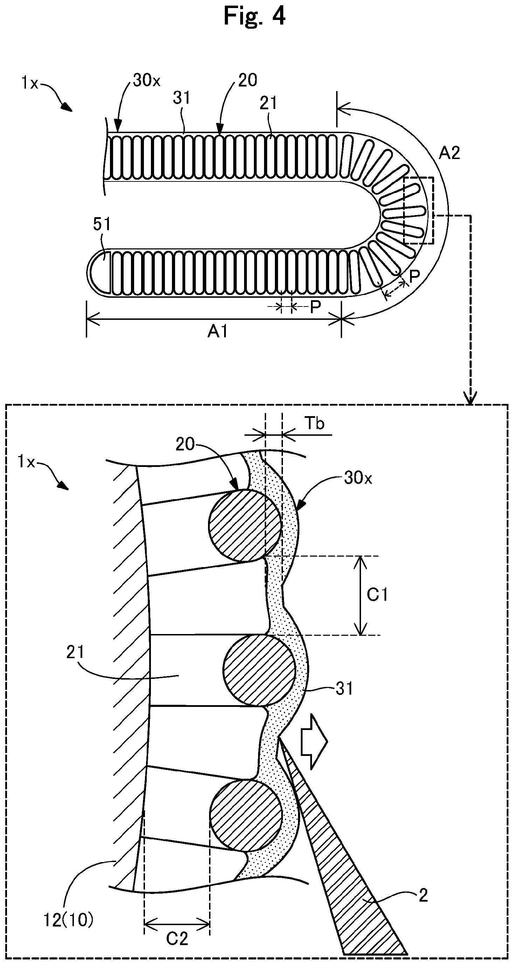

Fig. 4 is a diagram for explaining a resin layer 30x when a guide wire 1x in a comparative example is curved. In the guide wire 1x in the comparative example, the resin layer 30x is composed of only the surface layer 31 and does not include the protruding portion 32. In other words, in the guide wire 1x, the resin layer 30x is formed only on the outer peripheral surface of the coil body 20.

Thus, as illustrated in the lower side in Fig. 4, in the guide wire 1x, the thickness Tb while being elongated of the resin layer 30x cannot be kept sufficiently thick, and thus, if, for example, the distal end of the additional device 2 is caught on a surface of the resin layer 30x, sufficient resistance cannot be obtained against the force (lower side in Fig. 4: white arrow) in a direction in which the resin layer 30x is peeled off from the coil body 20. As a result, in the guide wire 1x in the comparative example, the resin layer 30x between the adjacent wires 21 may be peeled or damaged when the guide wire 1x is curved. In addition, the guide wire 1x in the comparative example does not include the protruding portion 32 (Figs. 2 and 3) extending toward the core shaft 10 relative to the inner peripheral surface of the coil body 20, and thus, if the core shaft 10 is bent inside the coil body 20, the core shaft 10 may come into contact with the coil body 20 to cause damage or deformation of the core shaft 10 and the coil body 20.

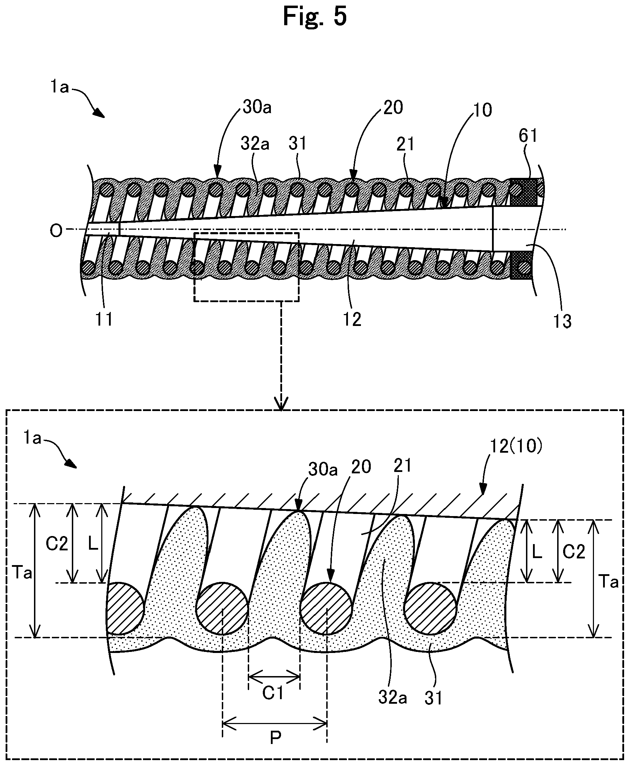

First embodiment of the invention Fig. 5 is a partial enlarged view of a guide wire 1a according to an embodiment of the invention. In the guide wire 1a according to the second embodiment, a resin layer 30a includes protruding portions 32a. Such a protruding portion 32a is formed so that a protrusion distal end of the protruding portion 32a (a portion closest to the core shaft 10) comes into contact with the core shaft 10. The shape of a protrusion of the protruding portion 32a in the cross section illustrated in Fig. 5 is an elliptical arc shape. The protruding portion 32a may be formed by using a material and manufacturing method similar to those of the protruding portion 32 according to the first embodiment.

The protruding length L of the protruding portion 32a in the guide wire 1a is equal to the size of the void C2 between the wire 21 and the core shaft 10. Thus, the thickness Ta of the resin layer 30a can be made thicker than that in the guide wire 1 according to the first embodiment. All of the protruding portions 32a included in the guide wire 1a may contact the core shaft 10, or at least some thereof may not contact the core shaft 10.

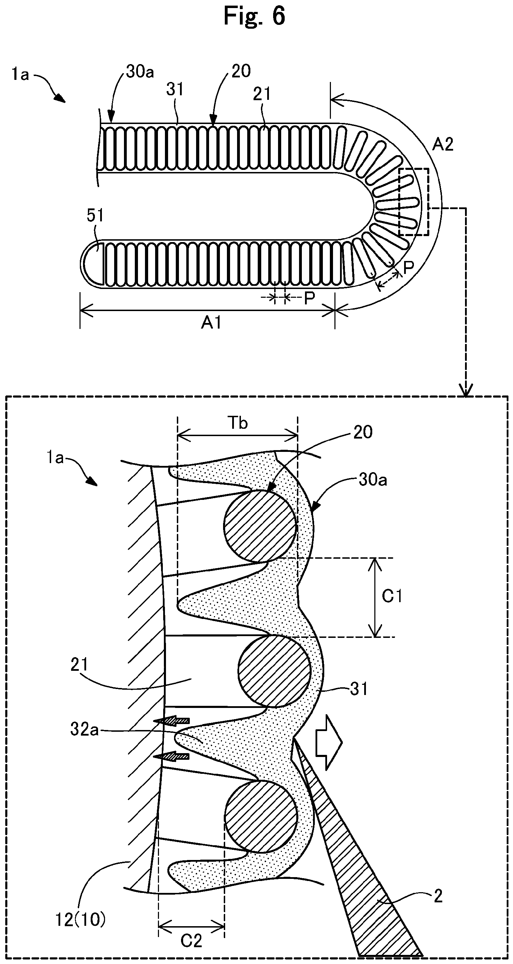

Fig. 6 is a diagram for explaining the resin layer 30a when the guide wire 1a according to the embodiment of the invention above is curved. The guide wire 1a according to the second embodiment has the thickness Ta that is thick enough to cause the resin layer 30a (protruding portion 32a) to come into contact with the core shaft 10 (Fig. 5). Thus, as illustrated in Fig. 6, the thickness Tb while being elongated of the resin layer 30a at a gap expanded with the curve of the guide wire 1a between the adjacent wires 21, can be kept much thicker.

Thus, if, for example, the distal end of the additional device 2 is caught on a surface of the resin layer 30a, larger resistance (lower side in Fig. 6: arrow with oblique hatching) can be obtained against the force (lower side in Fig. 6: white arrow) in a direction in which the resin layer 30a is peeled off from the coil body 20. Consequently, in the guide wire 1a according to the embodiment of the invention above, it is possible to further suppress peeling and damage of the resin layer 30a when the guide wire 1a is curved. Further, in the guide wire 1a according to the embodiment of the invention above, as in the first embodiment, the core shaft 10 is prevented from contacting with the coil body 20, and thus, damage of the core shaft 10 and the coil body 20 can be suppressed.

In addition, in the guide wire 1a according to the embodiment of the invention above, as in the first example, the void C2 is formed between the coil body 20 and the core shaft 10. Thus, as compared to a configuration having no voids C2, the wire 21 forming the coil body 20 easily moves, and thus, it is possible to reduce power required to curve the guide wire 1a and improve the flexibility of the guide wire 1a. Further, also in the guide wire 1a according to the embodiment of the invention above, the resin layer 30a can be easily formed as compared to a configuration having no voids C2.

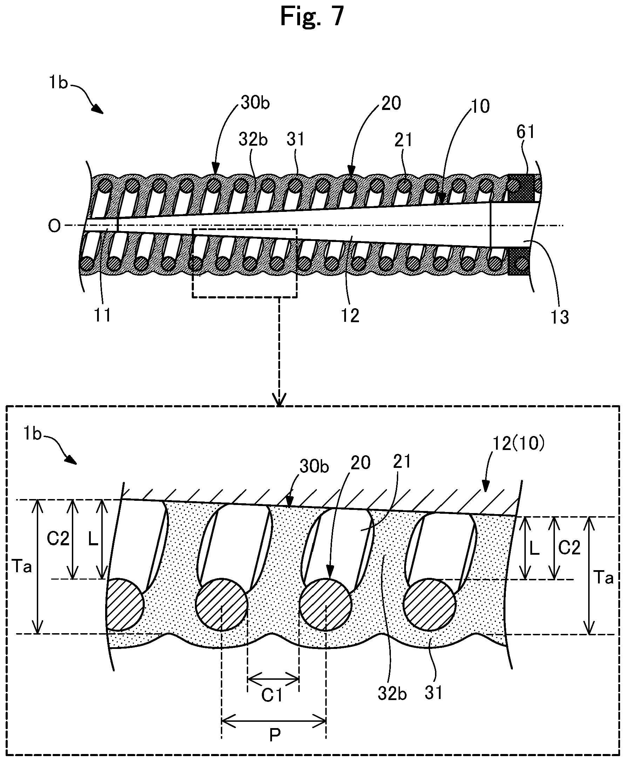

Second embodiment of the invention Fig. 7 is a partial enlarged view of a guide wire 1b according to a second embodiment of the invention. In the guide wire 1b according to this second embodiment, a resin layer 30b includes protruding portions 32b. A protrusion distal end of such a protruding portion 32b is joined to the core shaft 10. The shape of a protrusion of the protruding portion 32b in the cross section illustrated in Fig. 7 is a substantially rectangular shape in which a diameter of the center portion is reduced. The protruding portion 32b may be formed of a material similar to that of the protruding portion 32 according to the first example. The protruding portion 32b may be formed, for example, by applying an adhesive such as an epoxy-based adhesive to the outer peripheral surface of the core shaft 10 and then applying a liquidized resin material from the outer peripheral surface of the coil body 20. When the resin material is applied, the resin material is allowed to flow from the gap C1 of the coil body 20 to reach an adhesive layer of the core shaft 10. Thus, the protruding portion 32b joined to the core shaft 10 may be formed. Alternatively, fine irregularities may be provided on an outer surface of the core shaft 10, and then a molten resin material is poured toward the core shaft 10 and is physically joined to the core shaft 10 to form the protruding portion 32b.

The protruding length L of the protruding portion 32b in the guide wire 1b is equal to the size of the void C2 between the wire 21 and the core shaft 10. Thus, the thickness Ta of the resin layer 30b can be made thicker than that in the guide wire 1 according to the first embodiment. All of the protruding portions 32b included in the guide wire 1b may be joined to the core shaft 10, or at least some thereof may not be joined to the core shaft 10.

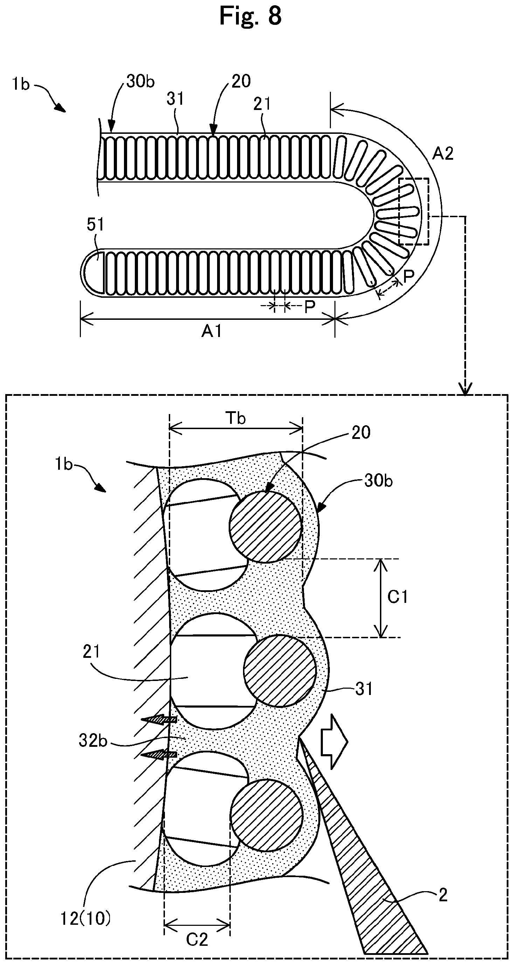

Fig. 8 is a diagram for explaining the resin layer 30b when the guide wire 1b according to the second embodiment of the invention. is curved. In the guide wire 1b according to the second embodiment of the invention, the resin layer 30b (protruding portion 32b) is joined to the core shaft 10. Thus, as illustrated in Fig. 8, the thickness Tb while being elongated of the resin layer 30b at a gap expanded with the curve of the guide wire 1b, between the adjacent wires 21, can be made equal to the thickness Ta during a normal time (that is, the thickness Tb while being elongated can be kept much thicker).

Thus, if, for example, the distal end of the additional device 2 is caught on a surface of the resin layer 30b, larger resistance (lower side in Fig. 8: arrow with oblique hatching) can be obtained against a force (lower side in Fig. 8: white arrow) in a direction in which the resin layer 30b is peeled off from the coil body 20. Consequently, in the guide wire 1b according to the second embodiment of the invention, it is possible to further suppress peeling and damage of the resin layer 30b when the guide wire 1b is curved. Further, in the guide wire 1b according to the second embodiment of the invention, as in the first embodiment, the core shaft 10 is prevented from contacting with the coil body 20, and thus, damage of the core shaft 10 and the coil body 20 can be suppressed.

In addition, in the guide wire 1b according to the second embodiment of the invention, as in the first embodiment and the second embodiment, the void C2 is formed between the coil body 20 and the core shaft 10, and thus, as compared to a configuration having no voids C2, it is possible to improve the flexibility of the guide wire 1b and easily form the resin layer 30b.

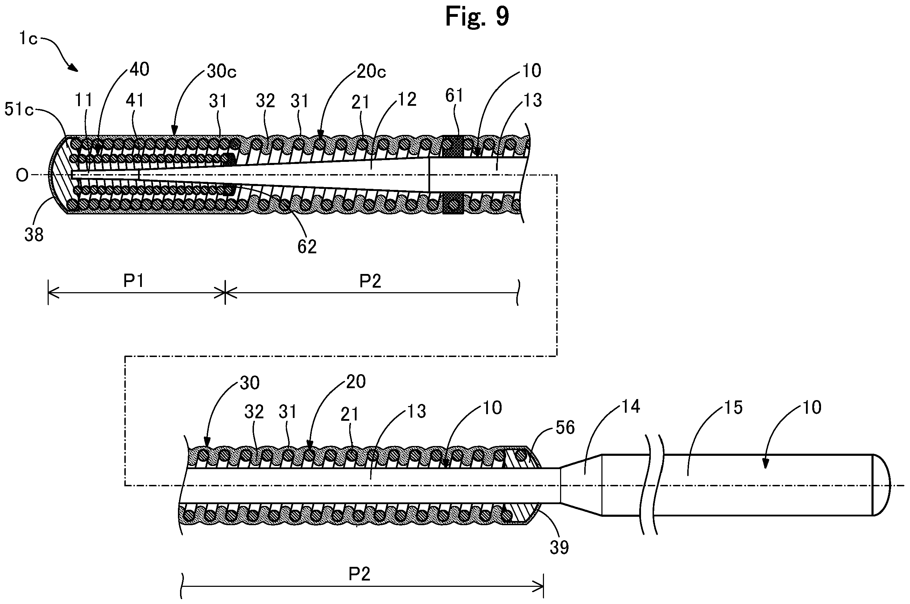

Second Example Fig. 9 is a schematic partial cross-sectional view illustrating an entire configuration of a guide wire 1c according to a second example. In the guide wire 1c according to the second example, a coil body 20c is formed, in a range P1 on the distal end side, as a closely wound part in which the adjacent wires 21 contact with each other without having the gap C1 (Fig. 2), and is formed, in a range P2 on the proximal end side from the range P1, as a sparsely wound part in which there is the gap C1 between the adjacent wires 21. Further, in the closely wound part (range P1) of the coil body 20c, the guide wire 1c includes, at an inner side of the coil body 20c, an inner coil body 40 formed by spirally winding a wire 41 around the core shaft 10. The inner coil body 40 has a length shorter than a length of the coil body 20c in the axis O direction.

A winding direction of the wire 41 in the inner coil body 40 is opposite to a winding direction in the coil body 20c. The winding direction of the wire 41 in the inner coil body may be the same as that in the coil body 20c, or may be a different direction other than the opposite direction. As with the wire 21, the wire 41 may be a solid wire or a strand. The inner coil body 40 may be configured by combining a single coil and a hollow stranded coil. A wire diameter of the wire 41 and an average coil diameter in the inner coil body 40 may be optionally determined. The wire 41 may be formed of a material similar to that of the wire 21 described in the first embodiment. The material used for the wire 41 may be the same as or different from that of the wire 21.

The inner coil body 40 is fixed to the core shaft 10 by a distal end joint part 51c and an inner fixing part 62. The distal end joint part 51c joins a distal end part of the coil body 20c, a distal end part of the inner coil body 40, and the distal end part of the small-diameter part 11 of the core shaft 10. The distal end joint part 51c may be formed of a material similar to that of the distal end joint part 51 described in the first example. The material employed for the distal end joint part 51c may be the same as or different from that of the distal end joint part 51. The inner fixing part 62 joins a proximal end part of the inner coil body 40 and a part of the first tapered part 12 of the core shaft 10. The inner fixing part 62 may be formed of a material similar to that of the distal end joint part 51 described in the first embodiment. The material employed for the inner fixing part 62 may be the same as or different from that of the distal end joint part 51.

As in the first example, a resin layer 30c includes the surface layer 31 and the protruding portion 32 in a portion (range P2) where the coil body 20c includes a sparsely wound part. On the other hand, in a portion (range P1) where the coil body 20c includes a closely wound part, the resin layer 30c includes only the surface layer 31 and does not include the protruding portion 32. Thus, the guide wire 1c may include, at a portion in the axis O direction, a closely wound part in which the adjacent wires 21 contact with each other without having the gap C1 (Fig. 2). A range in which the closely wound part is formed may be optionally determined. In the present embodiment, the closely wound part is formed at the distal end part of the coil body 20c (guide wire 1c). However, the disclosed example is not limited to this, and the closely wound part may be formed at a rear end part of the coil body 20c (guide wire 1c). In this case, the sparsely wound part may be formed at the distal end part of the coil body 20c (guide wire 1c), and the inner coil body 40 may be arranged at an inner side of the sparsely wound part of the coil body 20c. Further, the protruding portion 32 of the resin layer 30c may not be formed in the closely wound part of the coil body 20c.

The guide wire 1c according to the second example, can also exhibit a similar effect to that in the above-described first embodiment. With the guide wire 1c according to the second example, the sparsely wound part formed with the resin layer 30c is located in a portion (range P2) where the coil body 20c does not cover the inner coil body 40, and thus, the movement of the inner coil body 40 cannot be prevented by the protruding portion 32 of the resin layer 30c. Thus, according to the guide wire 1c of the present example, the flexibility of the guide wire 1c can be maintained even in the configuration including the inner coil body 40.

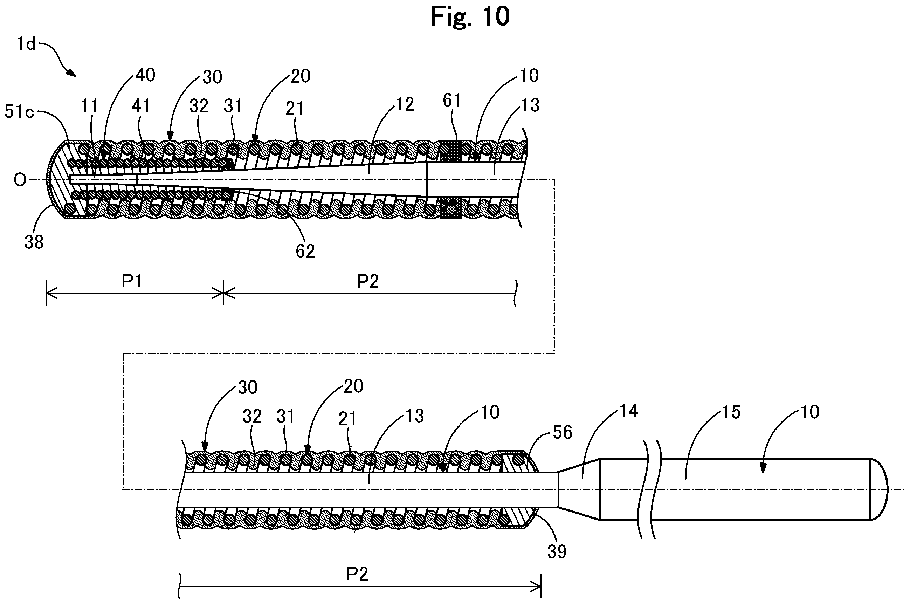

Third Example Fig. 10 is a schematic partial cross-sectional view illustrating an entire configuration of a guide wire 1d according to a third example. In the guide wire 1d according to the third example, the inner coil body 40 is provided at an inner side of the coil body 20, the entirety of which is formed as the sparsely wound part, in the range P1 on the distal end side. The inner coil body 40 has a length shorter than a length of the coil body 20 in the axis O direction. The configuration of the inner coil body 40 is similar to that of the second example. In Fig. 10, the inner coil body 40 is provided at the distal end part of the coil body 20 (guide wire 1d). However, the inner coil body 40 can be arranged at any position in the axis O direction. For example, the inner coil body 40 may be arranged at a center part of the coil body 20 or may be arranged at a rear end part of the coil body 20.

Thus, the sparsely wound part formed with the resin layer 30 may be located at a portion (range P1) where the coil body 20 covers the inner coil body 40. The guide wire 1d according to the fifth embodiment can also exhibit a similar effect to the above-described first embodiment and fourth embodiment.

### Third Embodiment of the Invention

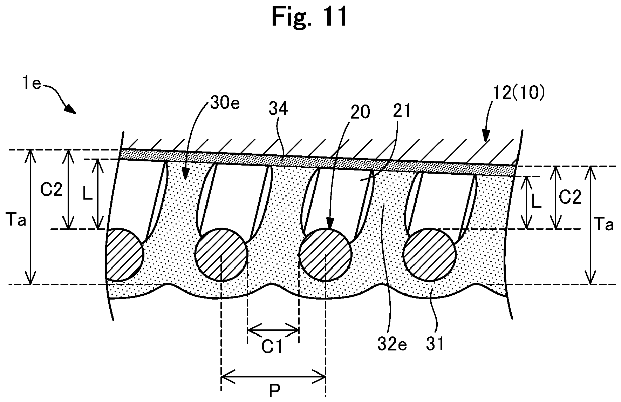

Fig. 11 is a partial enlarged view of a guide wire 1e according to a third embodiment. As with Fig. 2 described above, the left side in Fig. 11 corresponds to a distal end side (distal side) of the guide wire 1e and components, and the right side in Fig. 11 corresponds to a proximal end side (proximal side) of the guide wire 1e and components. The same applies to the following drawings.

In the guide wire 1e according to the third embodiment, a resin layer 30e includes protruding portions 32e and a resin film 34. The resin film 34 is a layer of resins that covers the outer peripheral surface (outer surface) of the core shaft 10. The resin film 34 may be formed of a material optionally selected from the resin materials listed for the resin layer 30 in the first embodiment. The resin film 34 may be formed, for example, by applying a liquidized resin material to the outer peripheral surface of the core shaft 10. The resin film 34 may be formed by joining a thin film-like resin material to the outer peripheral surface of the core shaft 10. For joining, for example, an adhesive such as an epoxy-based adhesive can be used. The thickness of the resin film 34 may be optionally determined.

A protrusion distal end of such a protruding portion 32e is joined to the resin film 34. The shape of a protrusion of the protruding portion 32e in the cross section illustrated in Fig. 11 is a substantially rectangular shape in which a diameter of the center portion is reduced. The protruding portion 32e may be formed of a material that is similar to that of the protruding portion 32 according to the first embodiment. The protruding portion 32e may be formed, for example, by applying an adhesive such as an epoxy-based adhesive to a surface of the resin film 34 and then applying a liquidized resin material from the outer peripheral surface of the coil body 20. When the resin material is applied, the resin material is allowed to flow from the gap C1 of the coil body 20 to reach an adhesive layer of the resin film 34. Thus, the protruding portion 32e joined to the resin film 34 may be formed. The protruding length L of the protruding portion 32e in the guide wire 1e is a length obtained by subtracting the thickness of the resin film 34 from the size of the void C2 between the wire 21 and the core shaft 10.

Thus, the thickness Ta of the resin layer 30e can be made thicker than that in the first embodiment. All of the protruding portions 32e included in the guide wire 1e may be joined to the resin film 34, or at least some thereof may not be joined to the resin film 34.

The guide wire 1e according to the third embodiment can also exhibit a similar effect to the above-described first example and second embodiment of the invention.

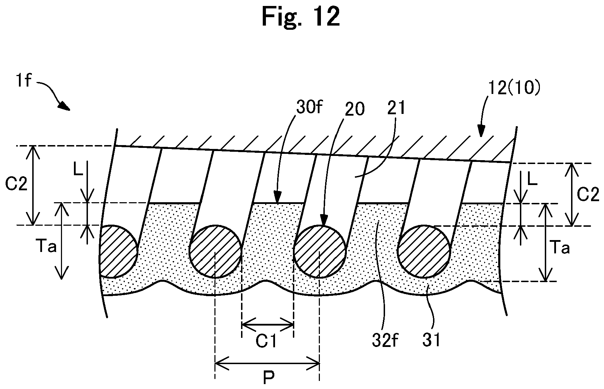

Fourth Example Fig. 12 is a partial enlarged view of a guide wire 1f according to a fourth example. In the guide wire 1f according to the fourth example, a resin layer 30f includes protruding portion 32f. The shape of such a protruding portion 32f in the cross section illustrated in Fig. 12 is a substantially rectangular shape. The protruding portion 32f may be formed of a material similar to that of the protruding portion 32 according to the first embodiment. For example, the protruding portion 32f may be formed as follows. First, a core material that is made of a fluorine-based resin and has a hollow cylindrical shape having an outer diameter larger than the outer diameter of the core shaft 10 is arranged at the inner side of the coil body 20, and in this state, a thin film-like resin material having flexibility is wound around the outer peripheral surface of the coil body 20. Next, the resin material is covered with a heat-shrinkable tube and then heated. As a result, the molten resin material flows into the core shaft 10 from the gap C 1 and comes into contact with the core material. Then, the core material is removed from the coil body 20 to form the protruding portion 32f having a substantially rectangular shape. Finally, the coil body 20 formed with the protruding portion 32f having a substantially rectangular shape is assembled to the core shaft 10. The protruding length L of the protruding portion 32f in the guide wire 1f may be optionally determined, as with the protruding portion 32 of the first example.

Thus, the protruding portion 32f can employ various shapes as long as the protruding portion 32f extends toward the core shaft 10 through the gap C 1 between the adjacent wires 21 of the coil body 20, and extends toward the core shaft 10 relative to the inner peripheral surface of the coil body 20. The guide wire 1f according to the fourth example can also exhibit a similar effect to the above-described first example,

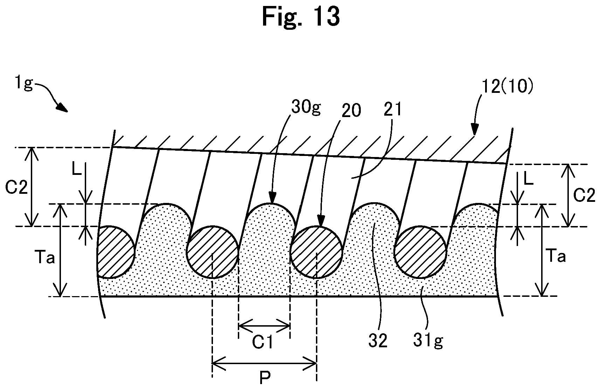

Fifth Example Fig. 13 is a partial enlarged view of a guide wire 1g according to an fifth example. In the guide wire 1g according to the eighth embodiment, a resin layer 30g includes a surface layer 31g. The shape of the surface layer 31g in the cross section illustrated in Fig. 13 is linear without any irregularities. The surface layer 31g may be formed of a material similar to that of the surface layer 31 according to the first example. For example, the surface layer 31g whose cross section is linear may be formed as follows. That is, a resin material having flexibility is wound around the outer peripheral surface of the coil body 20. Next, the resin material is covered with a heat-shrinkable tube having a flat outer surface (no irregularities) and then heated. As a result, the molten resin material flows into the core shaft 10 from the gap C1 to form the protruding portion 32 and the surface layer 31g whose cross section is linear. Finally, the coil body 20 formed with the protruding portion 32 and the surface layer 31g is assembled to the core shaft 10.

Thus, the surface layer 31g can function in various forms as long as the surface layer 31g covers at least the outer peripheral surface of the coil body 20. The guide wire 1g according to the fifth example can also exhibit a similar effect to the above-described first example.

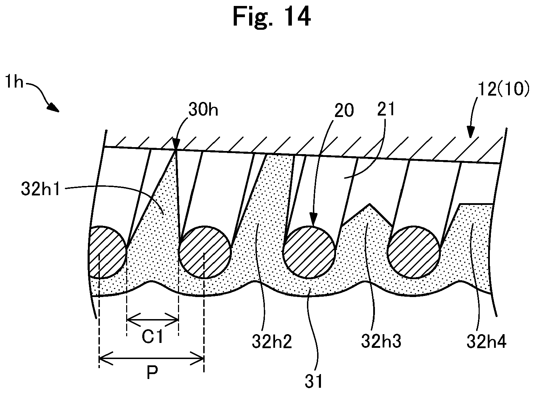

Fourth Embodiment of the Invention Fig. 14 is a partial enlarged view of a guide wire 1h according to a fourth embodiment. In the guide wire 1h according to the ninth embodiment, a resin layer 30h includes a protruding portion 32h1, a protruding portion 32h2, a protruding portion 32h3, and a protruding portion 32h4. The protruding portions 32h1 to 32h4 each have different shapes. In the cross section illustrated in Fig. 14, the shape of the protruding portion 32h1 is a substantially triangular shape, the shape of the protruding portion 32h2 is a trapezoidal shape, the shape of the protruding portion 32h3 is a substantially triangular shape having a height different from that of the protruding portion 32h1, and the shape of the protruding portion 32h4 is a trapezoidal shape having a height different from that of the protruding portion 32h2. The protruding portion 32h1 and the protruding portion 32h2 contact the core shaft 10. On the other hand, the protruding portion 32h3 and the protruding portion 32h4 do not contact the core shaft 10. The protruding portions 32h1 to 32h4 may be formed by using a material and manufacturing method similar to those of the protruding portion 32 of the first example and the protruding portion 32f of the fourth example.

Thus, the guide wire 1h may include various shapes of the protruding portions 32h1 to 32h4 as long as the protruding portions 32h1 to 32h4 extend toward the core shaft 10 through the gap C1 between the adjacent wires 21 of the coil body 20, and extend toward the core shaft 10 relative to the inner peripheral surface of the coil body 20. Further, the shapes of the protruding portions 32h1 to 32h4 may be different from each other as illustrated in Fig. 14. The guide wire 1h according to the fourth embodiment can also exhibit a similar effect to the above-described first embodiment.

### <Modification of Embodiment>

The disclosed embodiment is not limited to the above-described embodiments, and may be executed in various modes without departing from the spirit and scope of the disclosed embodiment. The following modifications can be applied, for example.

### [First Modification]

In the above-described examples and embodiments, configurations of the guide wire 1 have been described. However, the configuration of the guide wire 1 can be variously modified. For example, the guide wire 1 may have a configuration that does not include the second tapered part 14 and the second large-diameter part 15 (that is, a configuration in which the core shaft 10 is entirely covered with the coil body 20). For example, the guide wire 1 may be configured to not include a tapered part and have the same diameter over the entire region in the axis O direction.

### [Second Modification]

In the above-described examples and embodiments, configurations of the coil body 20 have been described. However, the configuration of the coil body 20 can be variously modified. For example, the coil body 20 may be arranged to cover the second tapered part 14 and the second large-diameter part 15 of the core shaft 10 in addition to the small-diameter part 11 to the first large-diameter part 13 of the core shaft 10 described above. For example, the arrangement of the closely wound part and the sparsely wound part of the coil body 20 may be optionally determined, and the closely wound part may be formed at a position other than the distal end part of the guide wire 1 (for example, at an almost center part in the axis O direction).

### [Third Modification]

In the above-described examples and iist embodiments, configurations of the resin layer 30 have been described. However, the configuration of the resin layer 30 can be variously modified. For example, at least one of the distal end resin layer 38 and the proximal end resin layer 39 included in the resin layer 30 may be omitted. For example, a configuration in which the resin layer 30 is not provided in the closely wound part of the coil body 20 (Fig. 9, fourth embodiment) may be employed. For example, the resin layer 30 may include the surface layer 31, the protruding portion 32 (Fig. 2) not contacting with the core shaft 10, and the resin film 34 (Fig. 11).

### [Fourth Modification]

The configurations of the guide wires of the examples and embodiments and the configurations of the guide wires of the first to third modifications may be appropriately combined. For example, the protruding portion 32 of the first example (Fig. 2, the configuration in which the protruding portion 32 does not contact the core shaft 10) may be adopted for a first region in the axis O direction, the protruding portion 32a of the first embodiment of the invention (Fig. 5, the configuration in which the protruding portion 32a contacts the core shaft 10) may be adopted for a second region, and the protruding portion 32b of the second embodiment of the invention, (Fig. 7, the configuration in which the protruding portion 32b is joined to the core shaft 10) may be adopted for a third region. In this case, the positions of the first to third regions may be optionally determined in consideration of the performance (slidability, thrombus adhesion preventing property, operability, etc.) required for the guide wire 1. Further, some of the first to third regions may be omitted, and the first and second regions may constitute the guide wire 1, the first and third regions may constitute the guide wire 1, or the second and third regions may constitute the guide wire 1.

### DESCRIPTION OF REFERENCE NUMERALS

1, 1a to 1h ... Guide wire
1x ... Guide wire in comparative example
2 ... Additional device
10 ... Core shaft
11 ... Small-diameter part
12 ... First tapered part
13 ... First large-diameter part
14 ... Second tapered part
15 ... Second large-diameter part
20, 20c ... Coil body
21 ... Wire
30, 30a to 30h ... Resin layer
31, 31g ... Surface layer
32, 32a to 32f, 32h1 to 32h4 ... Protruding portion
34 ... Resin film
38 ... Distal end resin layer
39 ... Proximal end resin layer
40 ... Inner coil body
41 ... Wire
51, 51c ... Distal end joint part
56 ... Proximal end joint part
61 ... Intermediate fixing part
62 ... Inner fixing part

## Claims

1. A guide wire(1a- 1e, 1h) comprising:
a core shaft(10);
a coil body(20,20c) formed by winding a wire around the core shaft(10); and
a resin layer(30a,30b,30e,30h) covering at least an outer peripheral surface of the coil body(20,20c), wherein
the coil body(20,20c) includes a sparsely wound part in which a gap is formed between adjacent wires,
a void is formed between the coil body(20,20c) and the core shaft(10), and
the resin layer(30a,30b,30e,30h) includes a protruding portion(32a,32b,32e,32h1,32h2) protruding toward the core shaft through the gap between the adjacent wires in the sparsely wound part, and the protruding portion(32a,32b,32e,32h1,32h2) extends toward the core shaft(10) relative to an inner peripheral surface of the coil body(20,20c), **characterized in that**
the protruding portion(32a,32b,32e,32h1,32h2) contacts the core shaft(10).

2. The guide wire(1b) according to claim 1, wherein
the protruding portion(32b) is joined to the core shaft(10).

3. The guide wire(1e) according to claim 1, further comprising:
a resin film(34) covering an outer peripheral surface of the core shaft(10), wherein
the protruding portion(32e) is joined to the resin film(34).

4. The guide wire(1c) according to any one of claims 1 to 3, further comprising:
an inner coil body(40) formed by winding a wire around the core shaft(10), at an inner side of the coil body(20c), wherein
the inner coil body(40) has a length shorter than a length of the coil body(20c) in an axial direction, and
the sparsely wound part is formed at a portion where the coil body(20c) does not cover the inner coil body(40).

## Patentansprüche

1. Führungsdraht (1a-1e, 1h), aufweisend:
einen Kernschaft (10);
einen Spulenkörper (20, 20c), der durch Wickeln eines Drahtes um den Kernschaft (10) gebildet ist; und
eine Harzschicht (30a, 30b, 30e, 30h), die mindestens eine äußere Umfangsfläche des Spulenkörpers (20, 20c) bedeckt, wobei
der Spulenkörper (20, 20c) einen spärlich gewickelten Teil umfasst, in dem eine Lücke zwischen benachbarten Drähten ausgebildet ist,
zwischen dem Spulenkörper (20, 20c) und dem Kernschaft (10) ein Hohlraum ausgebildet ist, und
die Harzschicht (30a, 30b, 30e, 30h) einen vorstehenden Abschnitt (32a, 32b, 32e, 32h1, 32h2) umfasst, der durch die Lücke zwischen den benachbarten Drähten in dem spärlich gewickelten Teil in Richtung des Kernschaft vorsteht, und der vorstehende Abschnitt (32a, 32b, 32e, 32h1, 32h2) sich in Richtung des Kernschafts (10) relativ zu einer inneren Umfangsfläche des Spulenkörpers (20, 20c) erstreckt, **dadurch gekennzeichnet, dass**
der vorstehende Abschnitt (32a, 32b, 32e, 32h1, 32h2) den Kernschaft (10) berührt.

2. Führungsdraht (1b) nach Anspruch 1, wobei
der vorstehende Abschnitt (32b) mit dem Kernschaft (10) verbunden ist.

3. Führungsdraht (1e) nach Anspruch 1, der ferner aufweist:
einen Harzfilm (34), der eine äußere Umfangsfläche des Kernschafts (10) bedeckt, wobei
der vorstehende Abschnitt (32e) mit dem Harzfilm (34) verbunden ist.

4. Führungsdraht (1c) nach einem der Ansprüche 1 bis 3, der ferner aufweist:
einen inneren Spulenkörper (40) an einer Innenseite des Spulenkörpers (20c), der durch Wickeln eines Drahtes um den Kernschaft (10) gebildet ist, wobei
der innere Spulenkörper (40) eine Länge aufweist, die kürzer ist als eine Länge des Spulenkörpers (20c) in einer axialen Richtung, und
der spärlich gewickelte Teil an einem Abschnitt ausgebildet ist, wo der Spulenkörper (20c) den inneren Spulenkörper (40) nicht bedeckt.

## Revendications

1. Fil-guide (1a-1e, 1h) comprenant :
une tige centrale (10) ;
un corps de bobine (20, 20c) formé en enroulant un fil autour de la tige centrale (10) ; et
une couche de résine (30a, 30b, 30e, 30h) recouvrant au moins une surface périphérique externe du corps de bobine (20, 20c), dans lequel
le corps de bobine (20, 20c) inclut une partie peu enroulée dans laquelle un espace est formé entre des fils adjacents,
un vide est formé entre le corps de bobine (20, 20c) et la tige centrale (10), et
la couche de résine (30a, 30b, 30e, 30h) inclut une partie saillante (32a, 32b, 32e, 32h1, 32h2) faisant saillie vers la tige centrale à travers l'espace entre les fils adjacents dans la partie peu enroulée, et la partie saillante (32a, 32b, 32e, 32h1, 32h2) s'étend vers la tige centrale (10) par rapport à une surface périphérique interne du corps de bobine (20, 20c), **caractérisé en ce que**
la partie saillante (32a, 32b, 32e, 32h1, 32h2) vient en contact avec la tige centrale (10).

2. Fil-guide (1b) selon la revendication 1, dans lequel
la partie saillante (32b) est reliée à la tige centrale (10).

3. Fil-guide (1e) selon la revendication 1, comprenant en outre :
un film de résine (34) recouvrant une surface périphérique externe de la tige centrale (10), dans lequel
la partie saillante (32e) est reliée au film de résine (34).

4. Fil-guide (1c) selon l'une quelconque des revendications 1 à 3, comprenant en outre
un corps de bobine interne (40) formé en enroulant un fil autour de l'arbre central (10), sur un côté interne du corps de bobine (20c), dans lequel
le corps de bobine interne (40) présente une longueur plus courte qu'une longueur du corps de bobine (20c) dans une direction axiale, et
la partie peu enroulée est formée au niveau d'une partie où le corps de bobine (20c) ne recouvre pas le corps de bobine interne (40).
